# EUROPÄISCHE PATENTANMELDUNG

(11) **EP 4 830 651 A1**
(43) Veröffentlichungstag der Anmeldung: **28.10.2026**
(21) Anmeldenummer: 25171754.2
(22) Anmeldetag: 22.04.2025
(51) Int. Cl.: A61B 18/04

(54) **PLASMASONDE UND SYSTEM**

(71) Anmelder: Erbe Elektromedizin GmbH, 72072 Tübingen (DE)
(72) Erfinder: SCHNITZLER, Uwe, 72072 Tuebingen (DE); SADLER, Daniel, 72074 Tuebingen (DE); BRANDT, Tjark, 72127 Kusterdingen (DE); SALKIC, Nermin, 72116 Mössingen (DE)
(74) Vertreter: Rüger Abel IP Legal Solutions GmbH

(57) **Zusammenfassung**

Die Erfindung betrifft eine Plasmasonde zur Erzeugung eines medizinisch einsetzbaren Plasmas, insbesondere zur Behandlung von Gewebe durch Koagulation. Die Sonde weist einen Sondenkörper auf, der sich von einem proximalen Sondenende zu einem distalen Sondenende erstreckt und mindestens ein zur Gasführung ausgebildetes Lumen umfasst. Am distalen Sondenende sind mindestens eine Elektrode und mindestens eine Gasaustrittsöffnung vorgesehen. Die Elektrode (36) umfasst einen Strömungsleitkörper, der zumindest abschnittsweise distal aus dem Sondenkörper herausragt und mit mindestens einer windschief zur Längsachse verlaufenden Leitfläche versehen ist. Die Leitfläche ist so ausgestaltet, dass der austretenden Gasströmung eine laterale Strömungskomponente überlagert wird, wodurch eine gezielte Formung und Aufweitung der Plasmazone ermöglicht wird. Dies verbessert insbesondere die Zündstabilität und die Wirksamkeit bei der seitlichen Anwendung. Die Erfindung umfasst ferner ein System aus der Plasmasonde und einer Versorgungseinrichtung mit einem Hochfrequenzgenerator und einer Gasquelle zur Plasmaerzeugung.

## Beschreibung

Die vorliegende Erfindung betrifft eine Plasmasonde zur Erzeugung eines insbesondere medizinisch einsetzbaren Plasmas. Das Plasma kann beispielsweise zur Oberflächenbehandlung von menschlichem Gewebe, vorzugsweise zur Koagulation von menschlichem Gewebe eines Patienten, eingesetzt werden. Darüber hinaus betrifft die Erfindung ein System bestehend aus einer derartigen Plasmasonde und einer zugehörigen Versorgungseinrichtung.

In der Medizin sind Plasmasonden zum Erzeugen eines Plasmas und Applizieren des Plasmas auf eine Gewebeoberfläche bereits in unterschiedlichen Ausführungen aus dem Stand der Technik bekannt.

In der EP 1 682 023 B1 wird eine Sonde zur Koagulation von Gewebe beschrieben, die zum Zuführen von Argongas in einen Raum zwischen einer Elektrode innerhalb der Sonde und dem Gewebe ausgebildet sind. Bei der Sonde steht die Elektrode mit einem distalen Ende aus der Sonde hervor. Am distalen Ende ist zudem eine Leiteinrichtung zum Richten und Leiten des Gases oder Plasmas derart angeordnet, dass wenigsten ein Teil des strömenden Gases oder Plasmas in die vorbestimmte Richtung umgeleitet wird.

Aus der JP 2002-301088 A ist eine Plasmasonde bekannt, bei der ein fluidführendes Lumen zu einer distalen Elektrode geführt ist. Innerhalb der Plasmasonde wird durch die Kombination aus Fluidstrom und Hochfrequenzstrom Plasma erzeugt. Das Plasma tritt anschließend über eine Düsenöffnung am distalen Ende der Sonde aus.

Aus der WO 2017/198869 A1 ist ein Plasmasystem bekannt, bei dem zwei Elektroden helixförmig um einen dielektrischen Körper gewickelt sind. Es handelt sich dabei insbesondere um Flachbandelektroden, die an einer Umfangsfläche des dielektrischen Körpers anliegen. In dem dielektrischen Körper sind Öffnungen vorgesehen, durch die das Gas zur Plasmaerzeugung in die Bereiche zwischen den Elektroden entweichen kann, in denen ein elektrisches Feld anliegt.

Die DE 696 33 182 T2 offenbart ferner einen Katheter mit einer spiralförmig verlaufenden Flachbandelektrode.

Die DE 11 2022 000 926 T5 beschreibt eine elektrochirurgische Vorrichtung mit einer Gegenelektrode und einer Aktivelektrode mit hydrophober oder superhydrophober Oberfläche. Diese ist so ausgelegt, dass sie einen Teil des Gases aus einer Dampftasche zurückhält, wenn sich die aktive Elektrode in einer leitfähigen, flüssigen Lösung befindet und in einen inaktiven Zustand übergegangen ist.

Aus der DE 100 30 111 A1 ist eine Sonde für die elektrochirurgische Behandlung von Gewebe bekannt, die eine rohr- oder schlauchförmige Zuleitung zum Transport eines Edelgases von einer Quelle zum distalen Ende sowie eine Elektrodeneinrichtung mit einem Entladungsabschnitt und einer elektrischen Zuleitung zum Zuführen von HF-Strom umfasst.

In bestimmten medizinischen Anwendungen, wie beispielsweise der Plasmakoagulation von schwer zugänglichen und verwinkelten Bereichen, wie z.B. dem Magen, stellt die Positionierung und Ausrichtung der Plasmasonde zum Gewebe des Patienten weiterhin eine Herausforderung dar.

Insbesondere beim monopolaren Betrieb der Plasmasonde - das bedeutet, dass die Plasmasonde eine Aktivelektrode aufweist und die Gegenelektrode entfernt am Körper des Patienten angebracht ist - muss ein präziser Abstand zwischen der Sonde und dem zu behandelnden Gewebe eingehalten werden. Nur unter diesen Bedingungen können die Hochfrequenzströme (HF-Ströme) zwischen Aktivelektrode und Gewebe innerhalb der Gasströmung fließen bzw. zünden und dabei Plasma erzeugen. Ein zu großer Abstand erschwert das Zünden bzw. Fließen der HF-Ströme zwischen Aktivelektrode und Gewebe, wodurch kein Plasma erzeugt wird und kein therapeutischer Effekt erzielt wird. Ein zu geringer Abstand hingegen erhöht das Risiko einer ungewollten Gewebekontaktierung mit der Aktivelektrode, was zu ungewollten Verletzungen des Gewebes führen kann. Bei den meisten bekannten Plasmasonden erfolgt der Hauptgasfluss in axialer Richtung, was die seitliche Behandlung erschwert, da in diesem Bereich tendenziell weniger Plasma erzeugt wird.

Davon ausgehend liegt der vorliegenden Erfindung die Aufgabe zugrunde, eine verbesserte Plasmasonde bereitzustellen, mit der größere Gewebeflächen mit Plasma behandelt werden können. Insbesondere soll eine verbesserte Zündfähigkeit der Plasmasonde bei unterschiedlichen Abständen sowie in verschiedenen Winkeln zwischen der Plasmasonde und dem Gewebe erreicht werden.

Diese Aufgabe wird durch eine Plasmasonde mit den Merkmalen des Anspruchs 1 sowie durch ein zugehöriges System gemäß Anspruch 14 gelöst.

Die erfindungsgemäße Plasmasonde weist einen Sondenkörper auf, der sich von einem proximalen Sondenende zu einem distalen Sondenende hin erstreckt. Der Sondenkörper ist vorzugsweise als flexibler Schlauch oder als flexibles Rohr ausgebildet.

In dem Sondenkörper ist mindestens ein Lumen ausgebildet, das zur Führung eines Gases, wie z.B. Argon, vorgesehen ist. An dem distalen Sondenende der Plasmasonde ist mindestens eine Elektrode angeordnet. Zudem ist an dem distalen Sondenende mindestens eine Gasaustrittsöffnung ausgebildet. Das mindestens eine Lumen mündet in diese Gasaustrittsöffnung, sodass eine Gasströmung aus der Sonde austreten kann.

Unter Plasma wird im Rahmen der Erfindung ein elektrisch leitfähiges, ionisiertes Gas verstanden, das insbesondere bei atmosphärischem Druck durch Anlegen eines Hochfrequenzfeldes erzeugt wird. Vorzugsweise handelt es sich bei dem verwendeten Gas um Argon, sodass mit der Plasmasonde Argonplasma erzeugt wird.

Insbesondere weist die mindestens eine Elektrode einen Strömungsleitkörper auf, der sich entlang einer Längsachse erstreckt und zumindest abschnittsweise distal aus dem Sondenkörper herausragt. Der Strömungsleitkörper besteht bevorzugt aus einem elektrisch leitfähigen Material. Der Strömungsleitkörper kann z.B. aus Metall bestehen, beispielsweise aus Edelstahl, Wolfram, Aluminium, Kupfer, Silber und dergleichen, oder aus Kohlenstofffasern oder sonstigen Carbon-Elementen, wie Graphit, Graphen und dergleichen. Der Strömungsleitkörper kann außerdem mit einem thermisch und/oder elektrisch leitfähigen Überzug versehen sein, wie z.B. Silber. Zumindest abschnittsweise kann der Strömungsleitkörper die mindestens eine Elektrode bilden. Die als Strömungsleitkörper ausgebildete Elektrode kann sowohl zur Formgebung der Gas- bzw. Plasmaströmung als auch zur Erzeugung des Plasmas selbst verwendet werden.

Der Strömungsleitkörper weist mindestens eine Leitfläche auf, die sich entlang einer Verlaufsrichtung erstreckt. Die Verlaufsrichtung der Leitfläche ist (stets) windschief zur Längsachse des Strömungsleitkörpers. Die Verlaufsrichtung ist dabei als die Richtung definiert, in der sich die (radial äußerste) Kante der Leitfläche in einer Seitenprojektion fortsetzt, wobei die Projektion radial zur Längsachse erfolgt. Vorzugsweise ist die Verlaufsrichtung der Leitfläche in jedem Punkt der Leitfläche windschief zur Längsachse, d.h. die Verlaufsrichtung schneidet die Längsachse nicht. Die Leitfläche erstreckt sich insbesondere spiralförmig um die Längsachse des Strömungsleitkörpers. Der radiale Abstand zwischen Leitfläche und Längsachse kann in distaler Richtung zumindest im Wesentlichen konstant bleiben, kontinuierlich ab- oder zunehmen. Die Verlaufsrichtung der Leitfläche steht insbesondere (an jeder Stelle) in einem Steigungswinkel zur Längsachse des Strömungsleitkörpers. Diese Geometrie ermöglicht eine gezielte Umlenkung und Formung der austretenden Gasströmung. Insbesondere erstreckt sich die Leitfläche von einem innerhalb des Sondenkörpers liegenden Abschnitt des Strömungsleitkörpers bis in den außerhalb des Sondenkörpers liegenden Abschnitt des Strömungsleitkörpers.

Bevor die Gasströmung auf die Leitfläche oder Leitflächen des Strömungsleitkörpers trifft, strömt diese zumindest im Wesentlichen in axialer Richtung, d.h. parallel zur Längsachse von proximal zu distal. Die Ausgestaltung der Elektrode als Strömungsleitkörper ermöglicht es, die Gasströmung zusätzlich lateral nach Außen umzulenken und damit aufzuweiten. In Abhängigkeit von der (axialen) Strömungsgeschwindigkeit kann der Durchmesser der austretenden Gas- bzw. Plasmaströmung variiert werden: Bei geringerer Strömungsgeschwindigkeit ist der Durchmesser des Gaskegels (Argonkegels) größer als bei höherer Geschwindigkeit. Ist eine größere Behandlungsfläche gewünscht, kann der Durchmesser des austretenden Gaskegels (Argonkegels) durch Reduktion der Strömungsgeschwindigkeit des Gases vergrößert werden.

Eine Besonderheit der vorliegenden Erfindung liegt in der windschief zur Längsachse der Plasmasonde und innerhalb der Gasströmung angeordneten Leitfläche. Durch die windschiefe Anordnung kann dem austretenden Gasstrom zusätzlich zur axialen Strömungskomponente eine laterale Strömungskomponente überlagert werden. Dies führt zu einer Aufweitung des Gasstrahls in lateraler Richtung und damit zu einer breiteren Plasmaausbildung.

Dies ist insbesondere bei der Behandlung größerer Gewebeflächen oder bei einer seitlichen Applikation vorteilhaft. Darüber hinaus strömt die Gasströmung an der (radial äußeren) Kante der Leitfläche vorbei, an der die Hochfrequenz-Feldstärke konzentriert ist. Dadurch kann eine verbesserte seitliche Plasmaerzeugung an den (äußeren) Kanten der Leitflächen erreicht werden. Auf diese Weise kann insbesondere die seitliche Zündstabilität des Plasmas verbessert werden.

Der Strömungsleitkörper weist vorzugsweise mindestens eine (gewindeartige) Strömungsnut auf. Die (gewindeartige) Strömungsnut weist insbesondere zumindest abschnittweise einen gleichbleibenden und/oder zunehmenden und/oder abnehmenden Windungsradius zu der Längsachse in distaler Richtung auf. Es wird bevorzugt, dass der Windungsradius der Strömungsnut von dem proximalen Ende zum distalen Ende des Strömungsleitkörpers hin abnimmt. Die Leitfläche(n) wird/werden dabei vorzugsweise jeweils durch die Flanke der Strömungsnut gebildet, auf die die (axiale) Gasströmung trifft, d.h. durch die der Gasströmung zugewandten Flanke. Die Strömungsnut bildet vorzugsweise zusammen mit dem Sondenkörper, also der Ummantelung des Schlauchs bzw. Rohrs, die jeweilige Gasaustrittsöffnung.

Die Kanten der Strömungsnut, insbesondere der Übergang zwischen der Flanke der Strömungsnut und der übrigen Mantelfläche des Strömungsleitkörpers, sind vorzugsweise scharfkantig bzw. spitzkantig ausgestaltet. Diese spitz ausgebildeten Bereiche bewirken eine lokale Erhöhung der elektrischen HF-Feldstärke, wodurch die Zündung des Plasmas gezielt an diesen Stellen initiiert werden kann.

Nach erfolgter Zündung erfolgt die Führung und Aufrechterhaltung der Plasmaentladung entlang der jeweiligen Leitfläche, also entlang der der Gasströmung zugewandten Flanke der Strömungsnut. Da der Strömungsleitkörper gleichzeitig als Elektrode ausgebildet ist, wird das entlang der Flanke strömende Plasma kontinuierlich mit Hochfrequenzstrom versorgt, wodurch die Plasmaentladung stabilisiert und verlängert werden kann.

In einer bevorzugten Ausführungsform sind zwei, drei oder mehr Gasaustrittsöffnungen am distalen Sondenende der Plasmasonde ausgebildet. Entsprechend weist der Strömungsleitkörper in dieser Ausführungsform zwei, drei oder mehr Leitflächen auf, die jeweils einer Gasaustrittsöffnung zugeordnet und derart angeordnet sind, dass die Gasströmung auf die jeweiligen Leitflächen trifft. Hierdurch kann eine gleichmäßigere Gasverteilung erreicht werden.

Bei mehreren Gasaustrittsöffnungen weist der Strömungsleitkörper entsprechend mehrere Strömungsnuten auf - beispielsweise zwei, drei oder mehr -, die in der Mantelfläche des Strömungsleitkörpers ausgebildet sein können. Diese Strömungsnuten verlaufen vorzugsweise helixförmig bzw. schraubenlinienförmig um die Längsachse des Strömungsleitkörpers. In diesen Fällen können die Strömungsnuten gemeinsam mit der Ummantelung des Sondenkörpers mehrere Gasaustrittsöffnungen bilden.

Der Strömungsleitkörper weist einen innerhalb des Sondenkörpers angeordneten ersten Abschnitt und einen außerhalb des Sondenkörpers angeordneten zweiten Abschnitt auf. Die Leitflächen bzw. die Strömungsnuten können sich entlang eines Teilabschnitts, vorzugsweise jedoch entlang eines Großteils oder der gesamten Mantelfläche des Strömungsleitkörpers erstrecken. Sie können sowohl im ersten als auch im zweiten Abschnitt oder in beiden Bereichen verlaufen.

Die Querschnittsform der Strömungsnuten ist vorzugsweise asymmetrisch, insbesondere schlitzförmig, elliptisch oder nierenförmig. Die asymmetrische Ausgestaltung begünstigt eine gezielte Strömungsführung und verbessert die Plasmaverteilung.

Der Sondenkörper ist vorzugsweise als flexibler Schlauch ausgebildet. Der Strömungsleitkörper kann bei der Herstellung der Plasmasonde in den Schlauch eingeführt werden, wobei die Gasaustrittsöffnung(en) durch die Wand des Sondenkörpers und der Strömungsnut gebildet ist/sind. Dies erlaubt eine einfache und kosteneffiziente Herstellung der distalen Struktur der Plasmasonde.

Die Leitflächen verlaufen bevorzugt spiralförmig um die Längsachse des Strömungsleitkörpers. Bei mehreren Leitflächen sind diese gleichmäßig um die Umfangsfläche verteilt und verlaufen helixförmig. Dadurch ergibt sich eine gleichmäßige laterale Aufweitung des Gasstrahls beim Austritt.

Die geometrische Form des Strömungsleitkörpers kann im Wesentlichen zylindrisch, kegelstumpfförmig oder halbellipsoidisch ausgeführt sein. Dies ermöglicht eine anwendungsspezifische Optimierung der Strömungs- und Plasmaparameter.

Der Strömungsleitkörper bildet zumindest bereichsweise vorzugsweise vollständig die Elektrode, wodurch die Elektrode einerseits zur Umlaufweitung der Gasströmung und darüber hinaus zur Bereitstellung der HF-Ströme in der Gasströmung zur Erzeugung des Plasmas dienen kann.

In einer weiteren Ausgestaltung kann der Strömungsleitkörper einen zentralen Spülkanal umfassen, der zur Führung eines Spülfluids vorgesehen ist. Dieser verläuft vorzugsweise entlang der Längsachse des Strömungsleitkörpers und mündet in eine oder mehrere Spülöffnungen an einer Düse. Das Spülfluid kann beispielsweise eine NaCl-Lösung, Ringerlösung oder destilliertes Wasser sein.

Der Spülkanal ist hierzu mit einem separaten Lumen der Sonde verbunden, sodass das Spülfluid unabhängig von der Gasströmung vom proximalen zum distalen Sondenende transportiert werden kann.

Die Oberfläche des Strömungsleitkörpers weist vorzugsweise Vertiefungen auf. Vorzugsweise sind die Vertiefungen als eine Vielzahl von Nuten oder Einkerbungen ausgebildet. Die Tiefe der Vertiefungen sind vorzugsweise (wesentlich) kleiner als die Tiefe der Strömungsnuten. Die Vertiefungen führen dazu, dass die Oberfläche der Elektrode bzw. des Strömungskörpers zusätzliche Kanten aufweist, die zu einer lokalen Feldverstärkung führen und somit die Zündfähigkeit des Plasmas verbessern. Die Vertiefungen können auf der gesamten Oberfläche oder selektiv, insbesondere im Bereich außerhalb des Sondenkörpers, ausgebildet sein. Es wird bevorzugt, dass die Vertiefungen der Oberfläche jedoch ausschließlich an der Oberfläche des Strömungsleitkörpers und nicht in den Strömungsnuten ausgebildet sind.

Die Verlaufsrichtungen der Leitfläche bzw. der Leitflächen ist/sind in einem vorbestimmten Steigungswinkel gegenüber der Längsachse angeordnet. Der Steigungswinkel liegt insbesondere zwischen 90° und 0°, vorzugsweise zwischen 65° und 15°, besonders bevorzugt zwischen 65° und 35°.

Darüber hinaus kann die Leitfläche im Längsschnitt zumindest in einem Mittelbereich der Leitfläche eine Tangente aufweisen, die in einem vorbestimmten Anstellwinkel zur Radialrichtung des Strömungsleitkörpers angeordnet ist. Der Längsschnitt ist dabei ein Schnitt entlang der Längsachse durch den Strömungsleitkörper. Die Leitfläche kann zumindest teilweise eben oder zumindest teilweise gekrümmt sein. Im Längsschnitt kann die Leitfläche linear oder zumindest teilweise gekrümmt sein. Beispielsweise kann die Leitfläche im Längsschnitt konvex gekrümmt sein. Der Anstellwinkel kann insbesondere zwischen 0° und 90°, vorzugsweise zwischen 30° und 70°. Eine solche Anordnung kann die Richtung der Gasablenkung gezielt beeinflussen und die laterale Plasmaverteilung verbessern. Das lateral aus der Strömungsnut herausdrängende Gas kann so an der Kante der Strömungsnut vorbeistreichen, an der die elektrische HF-Feldstärke erhöht ist, wodurch die Zündfähigkeit des Plasmas verbessert werden kann.

Die Aufgabe der Erfindung wird darüber hinaus durch ein System aus einer Versorgungseinrichtung und einer Plasmasonde der obigen Art gelöst. Die zuvor beschriebenen Merkmale und Vorteile sind ebenso auf das erfindungsgemäße System anwendbar.

Die Versorgungseinrichtung umfasst einen HF-Generator zur Versorgung der Elektrode mit HF-Strömen sowie eine Gasquelle zur Bereitstellung eines Plasmagases, vorzugsweise eines Inertgases wie Argon. Die Strömungsgeschwindigkeit des Gases ist vorzugsweise regelbar. Der Begriff ''HF-Strom" bezeichnet im Rahmen der vorliegenden Anmeldung einen Wechselstrom mit einer Frequenz im Bereich von etwa 4 kHz bis 500 kHz, vorzugsweise zwischen 300 kHz und 500 kHz. Bei dem Gas handelt es sich insbesondere um ein Inertgas wie beispielsweise Argon. Die Strömungsgeschwindigkeit, mit der die Gasquelle die Gasströmung bereitstellt, ist vorzugsweise veränderbar.

Die Plasmasonde ist an die Versorgungseinrichtung angeschlossen, dass das mindestens eine Lumen mit der Gasquelle fluidisch verbunden ist und eine Gasströmung aus der mindestens eine Gasaustrittsöffnung ausgegeben wird. Die Elektrode ist elektrisch mit dem HF-Generator verbunden und kann so mit HF-Strömen beaufschlagt werden.

Die Plasmasonde ist insbesondere für den monopolaren Betrieb eingerichtet. Dabei ist der HF-Generator für einen monopolaren Betrieb ausgelegt, wobei die Elektrode der Plasmasonde die aktive Elektrode bildet und eine am Patientenkörper angebrachte Gegenelektrode vorgesehen ist. Die Aktivelektrode und die Gegenelektrode sind mit dem HF-Generator elektrisch verbunden. Es zeigen:
- Figur 1: ein Ausführungsbeispiel des erfindungsgemäßen Systems;
- Figur 2-6: Ausführungsbeispiele des distalen Sondenendes der Plasmasonde in einer teilweise geschnittenen Seitenansicht, darunter eine Ausführung mit zylindrischem, kegelstumpfförmigen und halbellipsoidischen Strömungsleitkörper, die teilweise innerhalb des Sondenkörpers angeordnet ist, und zylindrischen Strömungsleitkörper, der vollständig außerhalb des Sondenkörpers angeordnet ist;
- Figur 7-9: verschiedene Ausgestaltungen der Strömungsnut in Detailansicht, darunter U-, rechteckige und V-förmige Querschnittsformen;
- Figur 10: einen Querschnitt durch den Sondenkörper;
- Figur 11, 12: beispielhafte Ausgestaltungen des distalen Sondenendes der Plasmasonde in dreidimensionaler Ansicht;
- Figur 13: den Strömungsverlauf bei hoher Strömungsgeschwindigkeit;
- Figur 14: den Strömungsverlauf bei niedriger Strömungsgeschwindigkeit.

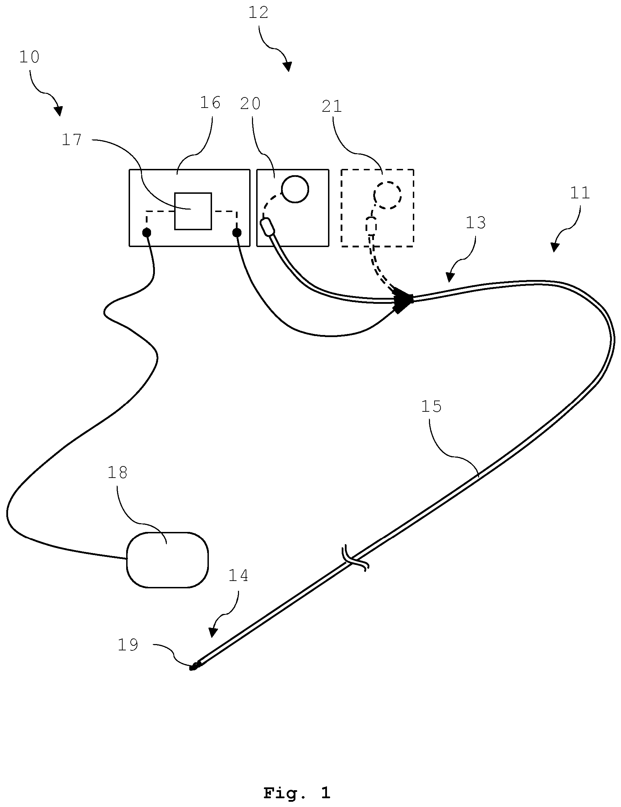

Figur 1 zeigt ein Beispiel des erfindungsgemäßen Systems 10 aus einer Plasmasonde 11 und einer Versorgungseinrichtung 12. Die Plasmasonde 11 erstreckt sich von einem proximalen Sondenende 13 zu einem distalen Sondenende 14. Sie weist einen Sondenkörper 15 auf, der aus einem flexiblen Rohr oder Schlauch gebildet ist.

Am proximalen Sondenende 13 besitzt die Plasmasonde 11 mehrere Anschlüsse, über die sie an die Versorgungseinrichtung 12 anschließbar ist.

Die Versorgungseinrichtung 12 weist eine HF-Einheit 16 mit einem HF-Stromgenerator 17 auf. Die HF-Einheit 16 besitzt einen Anschluss zur elektrischen Verbindung einer Gegenelektrode 18, die an einem Körperteil des Patienten angebracht werden kann. Zusätzlich ist ein Anschluss für die Plasmasonde 11 vorhanden, über den eine Zuleitung 19 zur Aktivelektrode angeschlossen werden kann. Die Zuleitung 19 leitet den HF-Strom vom proximalen Sondenende 13 zum distalen Sondenende 14, wo die Elektrode mit HF-Strom versorgt wird. Das gezeigte System 10 ist für den monopolaren Betrieb ausgelegt, bei dem die Elektrode der Plasmasonde 11 als Aktivelektrode fungiert und der Stromkreis über die Gegenelektrode 18 geschlossen wird.

Weiterhin umfasst die Versorgungseinheit 12 eine Gasquelle 20, die ein Plasmaerzeugungsgas, beispielsweise Argon, bereitstellt. Die Gasquelle 20 ist vorzugsweise so ausgebildet, dass die austretende Strömungsgeschwindigkeit einstellbar ist. Die Plasmasonde 11 ist über das proximale Sondenende 13 fluidisch mit der Gasquelle 20 verbunden.

Optional kann die Plasmasonde 11 außerdem dazu eingerichtet sein, am distalen Sondenende 14 ein Spülfluid auszugeben. Hierfür kann die Versorgungseinrichtung 12 eine Spülfluidquelle 21 aufweisen. Die Spülfluidquelle 21 ist in Figur 1 gestrichelt dargestellt, da diese rein optional ist. Die Spülfluidquelle 21 kann ein Spülfluid bereitstellen, bei dem es sich beispielsweise um eine Natriumchloridlösung, destilliertes Wasser oder eine Ringerlösung handeln kann.

Die HF-Einheit 16, die Gasquelle 20 und die Spülfluidquelle 21 können als separate Einheiten oder als eine kombinierte Einheit ausgebildet sein.

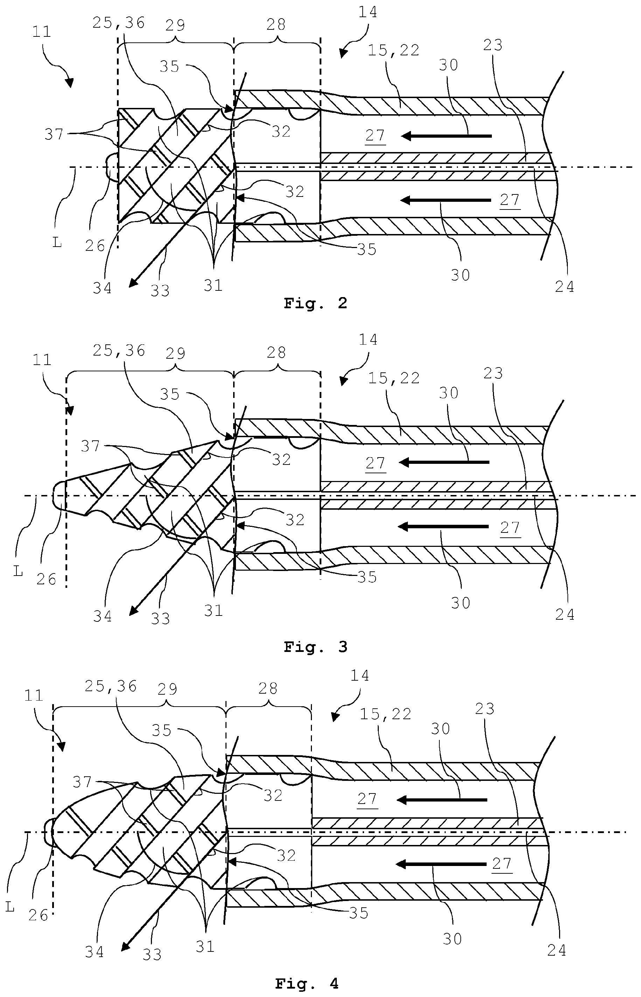

Figur 2 zeigt ein Beispiel der Ausgestaltung des distalen Sondenendes 14 der Plasmasonde 11 in einer teilweise geschnittenen Seitenansicht. Der Sondenkörper 15 der Plasmasonde 11 ist in den abgebildeten Beispielen als ein Schlauch oder flexibles Rohr mit einer Wand 22 ausgebildet. Die Wand 22 der Plasmasonde 11 kann aus einem flexiblen Kunststoffmaterial hergestellt sein. Ebenso kann die Wand 22 eine Verstärkungsstruktur aufweisen, die jedoch in den Figuren 2 bis 6 nicht eingezeichnet ist. In den in den Figuren 2 bis 6 gezeigten Beispielen ist die elektrische Zuleitung 23 zentrisch zur Wand 22 angeordnet abgebildet.

Bei den in den Figuren 2 bis 6 gezeigten Ausführungsbeispielen ist die elektrische Zuleitung 23 mit einem Spülfluidkanal 24 ausgebildet, der von dem proximalen Sondenende 13 bis zum distalen Sondenende 14 verläuft und am distalen Sondenende 14 in einen Strömungsleitkörper 25 mündet. Der Strömungsleitkörper 25 verlängert den Spülfluidkanal 24 bis hin zu einer distal angeordneten Düse 26, aus der das Spülfluid austreten kann.

Rund um die elektrische Zuleitung 23 ist ein Lumen 27 ausgebildet, durch welches die Gasströmung 30 vom proximalen zum distalen Sondenende 13, 14 geführt wird. Anders als in den Figuren 2 bis 6 abgebildet, können um die elektrische Zuleitung 23 herum auch mehrere Lumen ausgebildet sein, durch die beispielsweise mehrere Gasströmungen vom proximalen Sondenende zum distalen Sondenende transportiert werden können.

Der Strömungsleitkörper 25 ist in diesem Beispiel zylindrisch ausgeführt. Er weist einen ersten Abschnitt 28 auf, der innerhalb des Sondenkörpers 15 liegt, sowie einen zweiten Abschnitt 29, der distal aus dem Sondenkörper 15 herausragt. Der Strömungsleitkörper 25 erstreckt sich entlang einer Längsachse 30. Auf der Oberfläche des Strömungsleitkörpers 25 befinden sich Strömungsnuten 31, welche die auf den Strömungsleitkörper 25 treffende Gasströmung 30 umlenken. Die Strömungsnuten 31 weisen Leitflächen 32 auf, die sich entlang einer geneigten Verlaufsrichtung 33 erstrecken. Die Neigung zur Längsachse 30 wird durch einen Steigungswinkel 34 definiert.

Die Strömungsnuten 31 bilden zusammen mit der Wand 22 des Sondenkörpers 15 Gasaustrittsöffnungen 35, durch die die Gasströmung 30 austritt. Die Austrittsrichtung folgt dabei der Verlaufsrichtung 33 der jeweiligen Leitfläche 32. Dadurch kann sich die Gasströmung abhängig von ihrer Geschwindigkeit lateral ausbreiten.

Der Strömungsleitkörper 25 bildet die Elektrode 36. Diese wird über die elektrische Zuleitung 23 mit HF-Strom versorgt. Die Oberfläche des Strömungsleitkörpers 25 kann zusätzlich mehrere Vertiefungen 37 aufweisen, beispielsweise in Form von Quernuten, die quer zur Verlaufsrichtung 33 der Strömungsnuten 31 verlaufen. Die Vertiefungen 37 erzeugen zusätzliche Kanten, die zu einer lokalen Feldverstärkung und damit zu einer verbesserten Zündfähigkeit führen können. Die Vertiefungen 37 sind vorzugsweise nur im außerhalb des Sondenkörpers 25 (frei)liegenden Abschnitt 29 ausgebildet.

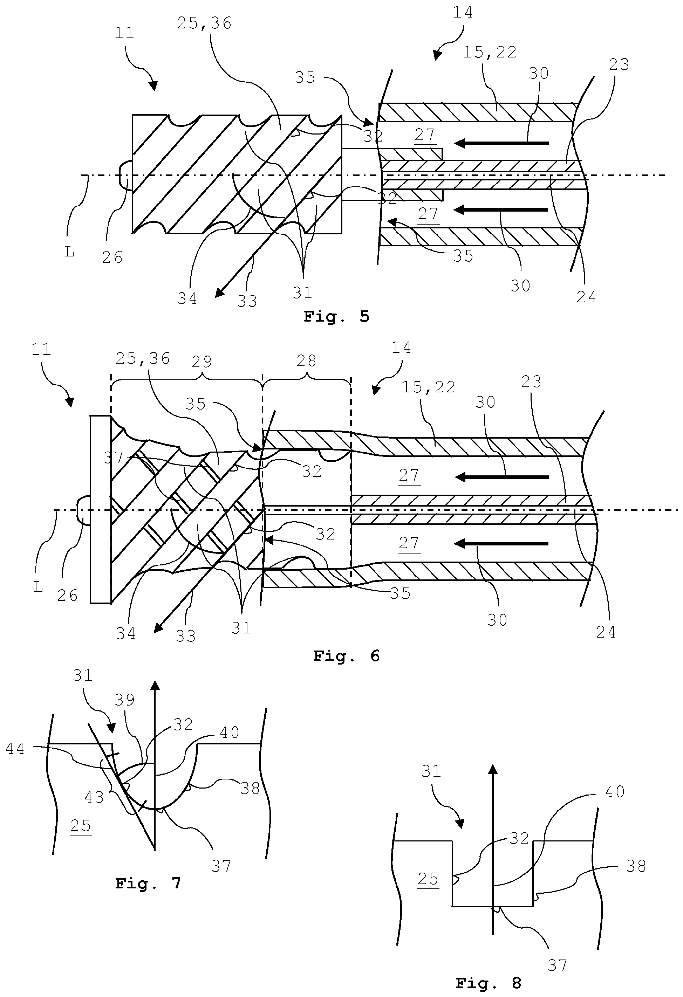

In den Figuren 3 bis 6 sind weitere Ausführungsformen des Strömungsleitkörpers 25 dargestellt. In Figur 3 ist der Strömungsleitkörper 25 kegelstumpfförmig und in Figur 4 halbellipsoidisch ausgebildet. In den in Figur 3 und 4 gezeigten Beispielen nimmt der Windungsradius der Strömungsnuten 31 von der axialen Höhe, an der die Gasaustrittsöffnungen 35 angeordnet sind, bis hin zum distalen Ende des Strömungsleitkörpers 25 ab. In Figur 5 ist der Strömungsleitkörper, wie in Figur 2, zylindrisch ausgebildet, allerdings vollständig außerhalb des Sondenkörpers 15 angeordnet. Um den Mündungsbereich, in welchem die Gasaustrittsöffnung 35 ausgebildet ist, kann wie in Figur 5 schematisch dargestellt eine Hülse angebracht sein, die die elektrische Zuleitung gegenüber der Gasströmung elektrisch isoliert. Die Hülse besteht insbesondere aus einem nicht elektrisch leitfähigen Material, wie z.B. aus Keramik. Figur 6 zeigt den Strömungsleitkörper 25 als Kegelstumpf, dessen Durchmesser in distaler Richtung hin zunimmt.

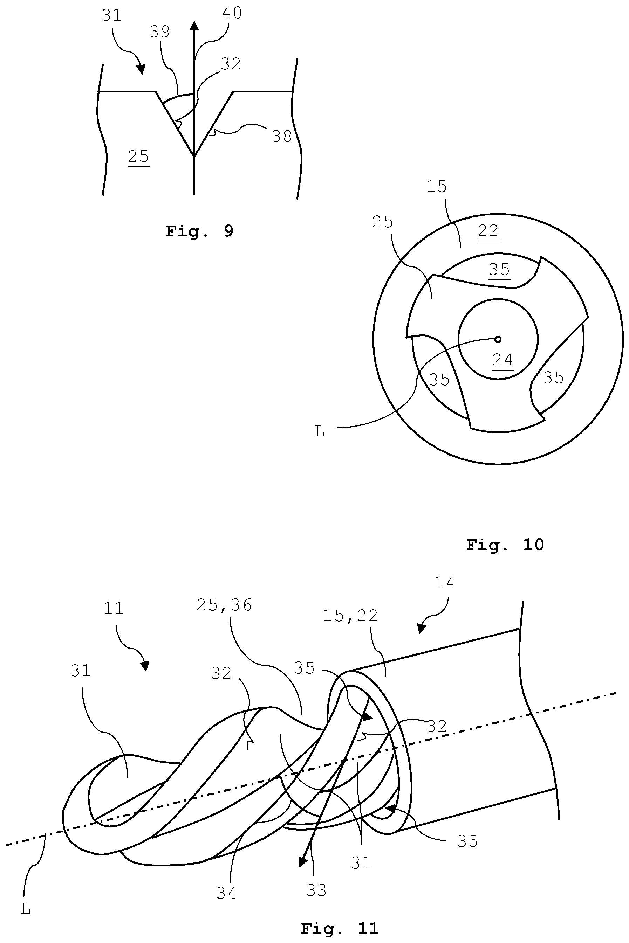

Die Figuren 7 bis 9 zeigen verschiedene Querschnitte der Strömungsnuten 31 durch den Strömungsleitkörper 25 in Detailansicht. Figur 7 zeigt eine U-förmige Nut mit einem Bodenabschnitt 37 am Scheitel der Strömungsnut 31 und einer gegenüberliegenden Flanke 38. Die Leitfläche 32 ist in diesem Beispiel konkav gekrümmt. Die Leitfläche 32 weist in einem Mittelbereich 43, der insbesondere weder die Kante noch den Scheitel der Strömungsnut 31 umfasst, eine Tangente 44 auf, die in einem Anstellwinkel 39 zur Radialrichtung 40 angeordnet ist. Figur 8 zeigt eine rechteckige Nutform. Die Leitfläche 32 ist in diesem Beispiel eben. In dem gezeigten Längsschnitt verläuft die Leitfläche 32 parallel zur Radialrichtung 40. Der Anstellwinkel ist in diesem Beispiel daher zumindest im Wesentlichen null. Figur 9 stellt eine V-förmige Nutform dar, bei der kein Bodenabschnitt vorhanden ist. Die Leitfläche 32 ist auch in diesem Beispiel zumindest im Wesentlichen eben.

Figur 10 zeigt einen Querschnitt durch das distale Sondenende 14. Die Wand 22 der Plasmasonde 11 ist insbesondere eine Schlauchwandung mit einem (kreis-)runden oder elliptischen Querschnitt. Die Gasaustrittsöffnungen 35 sind asymmetrisch und im Abstand zur Längsachse L angeordnet. Sie sind durch den Strömungsleitkörper 25 und die Wand 22 des Sondenkörpers 25 gebildet. Der Strömungsleitkörper 25 sitzt klemmend in der Wand 22 und besitzt einen größeren Durchmesser als dessen Innendurchmesser. In dem Strömungsleitkörper 25 ist außerdem ein Spülfluidkanal 24 ausgebildet, der entlang der Längsachse L von dem proximalen Ende des Strömungsleitkörpers 25 bis zum distalen Ende des Strömungsleitkörper 25 verlaufen und dort in eine Spüldüse 26 münden kann.

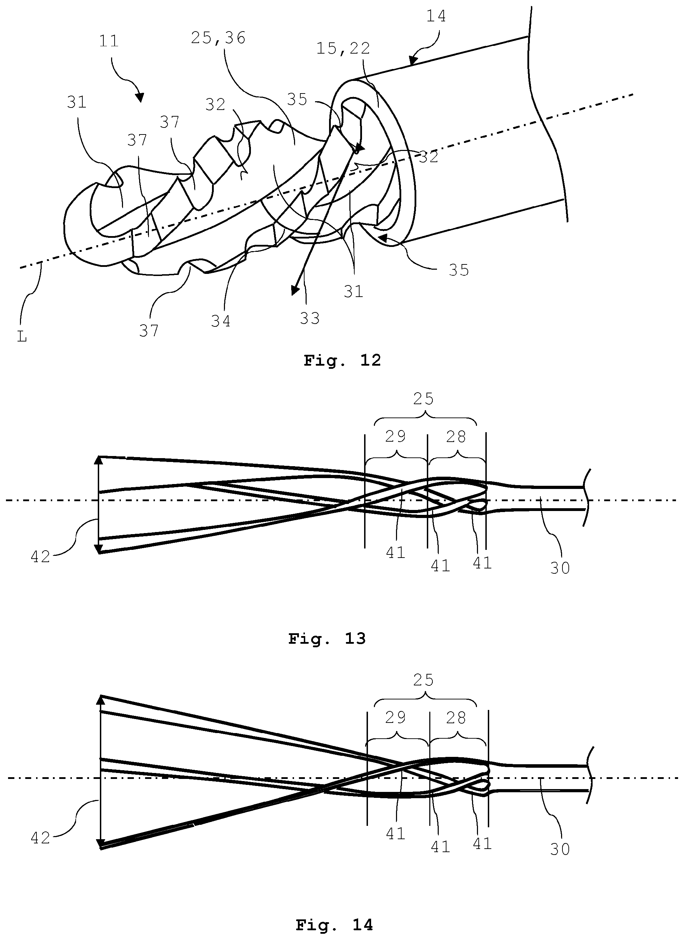

Die Figuren 11 und 12 zeigen perspektivische Ansichten des Strömungsleitkörpers 25. In Figur 11 sind spiralförmige Leitflächen 32 und mehrere Vertiefungen 37 auf der Oberfläche dargestellt. In Figur 12 sind die Vertiefungen 37 durch quer verlaufende Nuten gebildet. Zusätzlich können in den Strömungsleitkörpern 25, die in den Figuren 11 und 12 dargestellt sind, auch Spülfluidkanäle 24 ausgebildet sein, die an den distalen Enden der Strömungsleitkörper 25 in Spüldüsen 26 münden.

Figur 13 zeigt den Strömungsverlauf bei hoher Strömungsgeschwindigkeit der Gasströmung 30. Die Strömung trifft auf den ersten Abschnitt 28 des Strömungsleitkörpers 25 und wird in mehrere Teilgasströme 41 aufgeteilt, die entlang der Windung der Strömungsnuten 31 geführt werden. Die Strömung verlässt die Sonde über die Gasaustrittsöffnungen 35. Der Durchmesser 42 des Sprühkegels ist vergleichsweise klein.

Figur 14 zeigt die Strömungsverhältnisse bei niedriger Strömungsgeschwindigkeit der Gasströmung 30. Der Sprühkegeldurchmesser 42 ist hierbei größer als in Figur 13.

Die Erfindung betrifft eine Plasmasonde 11 zur Erzeugung eines medizinisch einsetzbaren Plasmas, insbesondere zur Behandlung von Gewebe durch Koagulation. Die Sonde 11 weist einen Sondenkörper 15 auf, der sich von einem proximalen Sondenende 13 zu einem distalen Sondenende 14 erstreckt und mindestens ein zur Gasführung ausgebildetes Lumen 27 umfasst. Am distalen Sondenende 14 sind mindestens eine Elektrode 36 und mindestens eine Gasaustrittsöffnung 35 vorgesehen. Die Elektrode 36 umfasst einen Strömungsleitkörper 25, der zumindest abschnittsweise distal aus dem Sondenkörper 15 herausragt und mit mindestens einer windschief zur Längsachse L verlaufenden Leitfläche 32 versehen ist. Die Leitfläche 32 ist so ausgestaltet, dass der austretenden Gasströmung 30 eine laterale Strömungskomponente überlagert wird, wodurch eine gezielte Formung und Aufweitung der Plasmazone ermöglicht wird. Dies verbessert insbesondere die Zündstabilität und die Wirksamkeit bei der seitlichen Anwendung. Die Erfindung umfasst ferner ein System 10 aus der Plasmasonde 11 und einer Versorgungseinrichtung 12 mit einem Hochfrequenzgenerator 17 und einer Gasquelle 20 zur Plasmaerzeugung.

### Bezugszeichenliste:

- 10: System aus Versorgungseinrichtung und Plasmakoagulationssonde
- 11: Plasmasonde
- 12: Versorgungseinrichtung
- 13: proximales Sondenende
- 14: distales Sondenende
- 15: Sondenkörper
- 16: HF-Einheit
- 17: HF-Stromgenerator
- 18: Gegenelektrode
- 19: Zuleitung für die Aktivelektrode
- 20: Gasquelle
- 21: Spülfluidquelle
- 22: Wand des Sondenkörpers
- 23: elektrische Zuleitung
- 24: Spülfluidkanal
- 25: Strömungsleitkörper
- 26: Düse
- 27: Lumen
- 28: erster Abschnitt
- 29: zweiter Abschnitt
- 30: Gasströmung
- 31: Strömungsnuten
- 32: Leitfläche
- 33: Verlaufsrichtung
- 34: Steigungswinkel
- 35: Gasaustrittsöffnung(en)
- 36: Elektrode(n)
- 37: Bodenabschnitt
- 38: gegenüberliegender Flächenabschnitt
- 39: Anstellwinkel
- 40: Radialrichtung
- 41: Teilgasströme
- 42: Durchmesser des austretenden Plasmas
- 43: Mittelbereich der Leitfläche
- 44: Tangente

- L: Längsachse

## Patentansprüche

1. Plasmasonde (11) mit einem Sondenkörper (15), der sich von einem proximalen Sondenende (13) zu einem distalen Sondenende (14) hin erstreckt und mindestens ein zur Gasführung ausgebildetes Lumen (27) aufweist, wobei an dem distalen Sondenende (14)
mindestens eine Gasaustrittsöffnung (35) ausgebildet ist, in die das Lumen (27) mündet und aus der eine Gasströmung (30) austreten kann, und
mindestens eine Elektrode (36) mit einem sich entlang einer Längsachse (L) erstreckenden Strömungsleitkörper (25), der zumindest abschnittsweise distal aus dem Sondenkörper (15) herausragt,
wobei der Strömungsleitkörper (25) mindestens eine in der Gasströmung (30) angeordnete Leitfläche (32) aufweist, die sich entlang einer Verlaufsrichtung (33) erstreckt, welche windschief zur Längsrichtung (L) verläuft.

2. Plasmasonde (11) nach Anspruch 1, **dadurch gekennzeichnet, dass** der Strömungsleitkörper (25) mindestens eine Strömungsnut (31) aufweist, wobei die Leitfläche (32) durch eine der Gasströmung (30) zugewandte Flanke (38) der Strömungsnut (31) gebildet ist.

3. Plasmasonde (11) nach Anspruch 1 oder 2, **dadurch gekennzeichnet, dass** der Strömungsleitkörper (25) einen innerhalb des Sondenkörpers (15) angeordneten ersten Abschnitt (28) und einen außerhalb des Sondenkörpers (15) angeordneten zweiten Abschnitt (29) aufweist.

4. Plasmasonde (11) nach einem der vorhergehenden Ansprüche, **dadurch gekennzeichnet, dass** die Strömungsnut (31) eine radiale Querschnittsfläche (z.B. 37, 38) aufweist, die vorzugsweise schlitzförmig, elliptisch, kreisrund oder nierenförmig ist.

5. Plasmasonde (11) nach einem der vorhergehenden Ansprüche, **dadurch gekennzeichnet, dass** der Sondenkörper (15) als, vorzugsweise flexibler, Schlauch gebildet ist, wobei der erste Abschnitt (28) des Strömungsleitkörpers (25) zusammen mit der Wandung (22) des Schlauchs die mindestens eine Gasaustrittsöffnung (35) bildet.

6. Plasmasonde (11) nach einem der vorhergehenden Ansprüche, **dadurch gekennzeichnet, dass** sich die Leitfläche (32) in einem spiralförmigen Verlauf um die Längsachse (L) des Strömungsleitkörpers (25) erstreckt.

7. Plasmasonde (11) nach einem der vorhergehenden Ansprüche, **dadurch gekennzeichnet, dass** der Strömungsleitkörper (25) aus elektrisch leitfähigem Material hergestellt ist.

8. Plasmasonde (11) nach einem der vorhergehenden Ansprüche, **dadurch gekennzeichnet, dass** der Strömungsleitkörper (25) zumindest bereichsweise, vorzugsweise vollständig, die Elektrode (36) bildet.

9. Plasmasonde (11) nach einem der vorhergehenden Ansprüche, **dadurch gekennzeichnet, dass** der Strömungsleitkörper (25) zumindest im Wesentlichen zylindrisch, kegelstumpfförmig oder halbellipsoidisch ausgebildet ist.

10. Plasmasonde (11) nach einem der vorhergehenden Ansprüche, **dadurch gekennzeichnet, dass** in dem Strömungsleitkörper (25) ein zur Führung eines Spülfluids eingerichteter Spülkanal (24) ausgebildet ist, der in eine Spüldüse (26) mit mindestens einer Spülöffnung mündet, durch die das Spülfluid ausgebbar ist, wobei der Spülkanal (24) vorzugsweise im Wesentlichen entlang der Längsachse (L) des Strömungsleitkörpers (25) verläuft.

11. Plasmasonde (11) nach einem der vorhergehenden Ansprüche, **dadurch gekennzeichnet, dass** an der Oberfläche (25) des Strömungsleitkörpers mehrere Vertiefungen (37) ausgebildet ist.

12. Plasmasonde (11) nach einem der vorhergehenden Ansprüche, **dadurch gekennzeichnet, dass** die Verlaufsrichtung (33) der Leitfläche (32) in einem vorbestimmten Steigungswinkel (34) gegenüber der Längsachse (L) angeordnet ist.

13. Plasmasonde (11) nach einem der vorhergehenden Ansprüche, **dadurch gekennzeichnet, dass** die Leitfläche (32) im Längsschnitt zumindest in einem Mittelbereich (43) der Leitfläche (32) eine Tangente (44) aufweist, die in einem vorbestimmten Anstellwinkel (39) zur Radialrichtung (40) des Strömungsleitkörpers (25) angeordnet ist.

14. System (10) aus einer Versorgungseinrichtung (12) und einer Plasmasonde (11) nach einem der vorhergehenden Ansprüche, wobei die Versorgungseinrichtung (12) aufweist:
- einen HF-Generator (17) zur Versorgung der Elektrode (36) mit HF-Strömen, und
- eine Gasquelle (20) zur Bereitstellung eines Gases zur Plasmaerzeugung, wobei die Strömungsgeschwindigkeit vorzugsweise veränderbar ist,
**dadurch gekennzeichnet, dass** die Plasmasonde (11) an die Versorgungseinrichtung (12) derart angeschlossen ist, dass das mindestens eine Lumen (27) mit der Gasquelle (20) fluidisch verbunden ist und eine Gasströmung (30) aus der mindestens einen Gasaustrittsöffnung (35) ausgebbar ist, und dass die Elektrode (36) elektrisch mit dem HF-Generator (17) verbunden ist und mit HF-Strömen beaufschlagt werden kann.

15. System (10) nach Anspruch 14, **dadurch gekennzeichnet, dass** der HF-Generator (17) für einen monopolaren Betrieb ausgelegt ist, wobei die Elektrode (36) der Plasmasonde (11) die Aktivelektrode (19) bildet und eine am Patientenkörper angebrachte Gegenelektrode (18) vorgesehen ist, die mit dem HF-Generator (17) elektrisch verbunden sind.
